(19) Europäisches Patentamt
European Patent Office
Office européen des brevets

(11) **EP 3 349 663 B1**

(12) **EUROPEAN PATENT SPECIFICATION**

(45) Date of publication and mention of the grant of the patent:
**05.12.2018 Bulletin 2018/49**

(21) Application number: **16763272.8**

(22) Date of filing: **12.09.2016**

(51) Int Cl.:
**A61B 8/08** (2006.01)

(86) International application number:
**PCT/EP2016/071391**

(87) International publication number:
**WO 2017/046019 (23.03.2017 Gazette 2017/12)**

(54) **ULTRASOUND APPARATUS AND METHOD FOR MEDICAL EXAMINATION OF A SUBJECT**

ULTRASCHALLVORRICHTUNG UND VERFAHREN ZUR MEDIZINISCHEN UNTERSUCHUNG EINER PERSON

APPAREIL À ULTRASONS ET PROCÉDÉ D'EXAMEN MÉDICAL D'UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.09.2015 EP 15185481**

(43) Date of publication of application:
**25.07.2018 Bulletin 2018/30**

(73) Proprietor: **Koninklijke Philips N.V.
5656 AE Eindhoven (NL)**

(72) Inventors:
- **KROON, Bart**
  **5656 AE Eindhoven (NL)**
- **MATHEW, Denny**
  **5656 AE Eindhoven (NL)**
- **VAN PUTTEN, Elbert Gerjan**
  **5656 AE Eindhoven (NL)**
- **LAMBERT, Nicolaas**
  **5656 AE Eindhoven (NL)**
- **KOLEN, Alexander Franciscus**
  **5656 AE Eindhoven (NL)**
- **BEZEMER, Rick**
  **5656 AE Eindhoven (NL)**

(74) Representative: **Veligura, Alina Viktorivna
Philips International B.V.
Intellectual Property & Standards
High Tech Campus 5
5656 AE Eindhoven (NL)**

(56) References cited:
EP-A1- 2 675 354          WO-A1-2013/181300
CN-A- 101 152 646         US-A1- 2010 234 716
US-A1- 2013 079 641       US-A1- 2013 237 822
US-A1- 2014 276 048

- TSAKALAKIS MICHAIL ET AL: "A wearable ultrasound multi-transducer array system for abdominal organs monitoring", 13TH IEEE INTERNATIONAL CONFERENCE ON BIOINFORMATICS AND BIOENGINEERING, IEEE, 10 November 2013 (2013-11-10), pages 1-5, XP032541218, DOI: 10.1109/BIBE.2013.6701592

## Description

FIELD OF THE INVENTION

The present invention relates to an ultrasound apparatus for medical examination of a subject. The present invention further relates to a method for medical examination of a subject.

BACKGROUND OF THE INVENTION

In the field of medical examination systems, ultrasound is a well-known and reliable technique for medical diagnostics, in particular to monitor hemodynamic or pulmonary parameters or to provide medical images of the patient.

For continuous monitoring of the patient, it is further known to attach one or a plurality of separate ultrasound transducers to the skin of the patient in order to determine measurement data in a non-invasive and non-obstructive way over a long-time frame continuously or frequently. A corresponding ultrasound apparatus for assessing the lung is e.g. known from US 5,485,841.

US 2013/0079641 A1, on which the preamble of claims 1, 13 and 15 is based, discloses a garment configured to be affixed to a portion of a living body, and at least one ultrasound transducer having a fixed position on the garment and configured to provide at least one of: produce and receive, ultrasound signals that pass through the living body.

A disadvantage of the known ultrasound monitoring systems is that the different transducers are complicated to wear and complicated to apply to the body. A further disadvantage is that the relative position of the ultrasound transducers is variable and the field of view of the transducers may be obstructed by an anatomical feature of the patient to be monitored.

SUMMARY OF THE INVENTION

It is therefore an object of the invention to provide an improved ultrasound apparatus for medical examination of a subject which is less complicated to wear and less complicated to apply and provides more reliable measurement data. It is further an object of the present invention to provide a corresponding improved method for medical examination of a subject.

According to one aspect of the present invention, an ultrasound apparatus for medical examination of a subject is provided comprising:

a plurality of ultrasound transducers for emitting and receiving ultrasound waves and for providing different ultrasound signals on the basis of the ultrasound waves,

a flexible and / or stretchable connection layer attachable to the subject, wherein the ultrasound transducers are coupled to the connection layer, and

a processing unit coupleable to the ultrasound transducers for receiving the ultrasound signals and for determining at least one parameter on the basis of the ultrasound signals derived from the ultrasound waves backscattered from an anatomical feature of the subject, wherein the at least one parameter is indicative of a position of the ultrasound transducers with respect to an anatomical feature of the subject and/or indicative of a medical condition of the subject wherein the processing unit is adapted to determine at least one parameter indicative of a relative position of the ultrasound transducers to each other and / or a shape of the connection layer,

wherein the ultrasound transducers (16) are adapted for inducing and measuring surface acoustic waves transmitted through the connection layer (18) or a layer attached to the connection layer (18), and wherein the the processing unit is adapted for determining a distance between the ultrasound transducers and/or the shape of the connection layer on the basis of the surface acoustic waves transmitted through the connection layer or a layer attached to the connection layer.

According to another aspect of the present invention a method for medical examination of a subject is provided comprising the steps of:

emitting and receiving ultrasound waves by means of a plurality of ultrasound transducers attachable (or attached) via a flexible and / or stretchable connection layer to the subject and providing a plurality of different ultrasound signals on the basis of the ultrasound waves received by the plurality of ultrasound transducers,

determining at least one parameter on the basis of the ultrasound signals received by the ultrasound transducers, said signals derived from the ultrasound waves backscattered from an anatomical feature of the subject, wherein the at least one parameter is indicative of a position of the ultrasound transducers with respect to an anatomical feature of the subject and / or indicative of a medical condition of the subject

determining at least one parameter indicative of a relative position of the ultrasound transducers to each other and / or a shape of the connection layer, and

determining a distance between the ultrasound transducers on the basis of the ultrasound waves received by the ultrasound transducers and/or determining the shape of the connection layer on the basis of the ultrasound waves received by the ultrasound transducers which are surface acoustic waves transmitted through the connection layer or a layer attached to the connection layer.

According to another aspect of the present invention a computer program product for medical examination of a subject is provided, the computer program product comprising computer-readable program code downloadable from a communications network, or storable on, or stored on a computer-readable storage medium, which computer-readable program code, when run on a computer causes the computer to perform the following a steps:

controlling a plurality of ultrasound transducers, for emitting and receiving ultrasound waves, attachable (or attached) via a flexible and / or stretchable connection layer to the subject and providing a plurality of different ultrasound signals on the basis of the ultrasound waves received by the plurality of ultrasound transducers,

determining at least one parameter on the basis of the ultrasound signals received by the ultrasound transducers, wherein the at least one parameter is indicative of a position of the ultrasound transducers with respect to an anatomical feature of the subject and / or indicative of a medical condition of the subject

determining at least one parameter indicative of a relative position of the ultrasound transducers to each other and / or a shape of the connection layer, and

determining a distance between the ultrasound transducers on the basis of the ultrasound waves received by the ultrasound transducers and/or determining the shape of the connection layer on the basis of the ultrasound waves received by the ultrasound transducers which are surface acoustic waves transmitted through the connection layer or a layer attached to the connection layer.

The computer program product can be suitable to work with a server device and client device including system. Part of the steps can be performed on the server device while the other or another part of the steps is performed on the client device. The server and client device can be remote from each other and connected through wired or wireless communication as known in the art. Alternatively, all steps are performed on a server device or on a client device.

In an embodiment, a computer system or device may be provided which comprises: a computer program product according to an embodiment of the present invention; and one or more processors adapted to perform a method according to an embodiment by execution of the computer-readable program code of said computer program product. The system or device can comprise a client device and server device, or either one of the two for execution of the method steps. The server and client device can have communication devices for communicating with each other using wired or wireless communication protocols.

The computer program product can have code for communicating results of any of the method steps to a user. Such code can include code for generating imagery, video and/or audio output. The system, client and/or server device can include a display and/or audio output devices (such as speakers) for generating the output.

The monitoring apparatus can in particular be used for monitoring the lung, the heart, the aorta, the carotid artery, the jugular vein, the brachial artery, and the femoral artery of the subject by means of the plurality of ultrasound transducers, which can be applied to the chest, the neck, the arm or the leg of the subject, respectively.

Preferred embodiments of the invention are defined in the dependent claims. It shall be understood that the claimed method has similar and/or identical preferred embodiments as the claimed device and as defined in the dependent claims.

The present invention is based on the idea to attach a plurality of ultrasound transducers by means of a connection or a contact layer to the subject to be examined in order to perform a non-invasive and non-obstructive continuous measurement, wherein the ultrasound transducers can be easily applied to the subject. Since the connection of the ultrasound transducers and the relative position of the ultrasound transducers to each other at the body of the subject can be varied individually and the field of view may be obstructed, the position of the ultrasound transducers with respect to the anatomical feature of the subject is determined on the basis of the ultrasound signals derived from the received ultrasound waves. Hence, an indication of an appropriate or inappropriate position of one or more of the ultrasound transducers can be provided to the user so that a more precise and more reliable monitoring and evaluation of the ultrasound data can be achieved.

The flexible and / or stretchable connection layer is preferably formed of a flexible and / or stretchable material. This is a possibility to individually connect the transducers to the subject with a high degree of freedom.

In an embodiment, the processing unit is adapted to provide an indication of the position of the ultrasound transducers with respect to an anatomical feature of the subject to a user. This is a possibility to provide a feedback of the quality of the position of the ultrasound transducers so that an obstruction of the ultrasound transducers can be avoided.

In a further embodiment, the position of the ultrasound transducers with respect to the anatomical feature is determined on the basis of at least one of A-line detection, amplitude and/or intensity detection or Doppler signal detection. This is a possibility to precisely detect anatomical features of the subject with low technical effort. This is in particular a possibility to determine obstructing anatomical features of the subject e.g. to determine a rib which can obstruct lung monitoring.

In a further embodiment, the processing unit is adapted to determine the at least one parameter indicative of the medical condition of the subject on the basis of B-line detection. This is a possibility to determine the medical condition of the subject precisely with low technical effort, wherein the B-line detection is can be utilized for detecting lung diseases like pulmonary edema or can be utilized for detecting diseases of the heart, the aorta, or an artery like cardiovascular diseases.

In a further embodiment, the ultrasound apparatus comprises an accelerometer, wherein the processing unit is adapted to determine a posture of the subject and / or changes of the posture of the subject on the basis of a signal received from the accelerometer. This is a possibility to improve the interpretation and evaluation of the ultrasound signals, since the posture of the subject is considered which is an influencing parameter for different medical conditions or parameters in particular hemodynamic or pulmonary parameters.

In a further embodiment, the ultrasound apparatus further comprises a plurality of height detectors associated to each of the ultrasound transducers for providing height signals corresponding to a height of the respective ultrasound transducers with respect to anatomical features of the subject, wherein the processing unit is adapted to determine a posture of the subject on the basis of the height signals. This is a further possibility to determine the posture of the subject which is an influencing parameter for different medical measurements in particular measurements like hemodynamic or pulmonary parameters. Further, the relative position of the ultrasound transducers with respect to gravity can be determined so that e.g. a fluid or a fluid height in the lung can be determined.

An ultrasound apparatus in accordance with the invention is defined in claim 1.

This is a possibility to improve the evaluation and the interpretation of the ultrasound signals since a registration of the ultrasound transducers can be performed. In particular a 2D-image or a 3D-image can be formed on the basis of the corresponding ultrasound signals and the relative position of the ultrasound transducers to each other. This is in general a possibility to improve the evaluation of the data which is comfortable for the user.

In a further embodiment, the ultrasound apparatus comprises a beam-forming unit for focusing the ultrasound waves emitted by the ultrasound transducers, wherein the beam-forming unit is adapted to focus the ultrasound waves emitted by the ultrasound transducers on the basis of the relative position of the ultrasound transducers to each other and/or on the basis of the position of the ultrasound transducers with respect to an anatomical feature of the subject. This is a possibility to modify the emitted ultrasound signals or waves based on the relative position of the ultrasound transducers to each other and/or based on the anatomical features of the subject so that the precision and the reliability of the measurement can be improved.

The processing unit is adapted to determine the distance between the ultrasound transducers on the basis of the ultrasound waves received by the ultrasound transducers. This is a possibility to determine the relative position of the ultrasound transducers to each other and to register the ultrasound transducers in the individual position at the subject, so that the evaluation of the ultrasound data can be improved and image data can be derived from the ultrasound signals.

Additionally or alternatively, the processing unit is adapted to determine a shape of the connection layer on the basis of the ultrasound waves received by the ultrasound transducers. This is a possibility to further improve the evaluation and interpretation of the ultrasound signal and to determine a 2D- or 3D-ultrasound image.

This is a possibility to determine the relative position of the ultrasound transducers with low technical effort even if the ultrasound transducers are not coupled via a direct ultrasound path.

In accordance with the invention, the ultrasound waves received by the ultrasound transducers for determining the distance between the ultrasound transducers and/or the shape of the connection layer are surface acoustic waves transmitted through the connection layer or an additional layer attached to the connection layer. This is a possibility to improve the robustness of the measurement of the relative position of the ultrasound transducers, since the distance and shape sensing depends only on the construction of the connection layer or the additional layer attached to the connection layer and not on the basis of the tissue characteristics of the subject.

The ultrasound waves for determining the distance between the ultrasound transducers and/or the shape of the connection layer are emitted by one of the ultrasound transducers. This is a possibility to determine the distance of the ultrasound transducers and/or the shape of the connection layer with low technical effort, since the integrated ultrasound transducers can be utilized also to determine the distances between the ultrasound transducers and/or the shape of the connection layer. The distances between the ultrasound transducers and the shape of the connection layer are determined by emitting one short ultrasound pulse by means of a single ultrasound transducer wherein the other transducers receive the pulse. The distance and the shape can be determined on the basis of the time of flight, the amplitude or the phase of the ultrasound pulse.

According to a further embodiment, the distance measurement is performed by emitting a short ultrasound pulse by a plurality of the ultrasound transducers or by each of the ultrasound transducers in a consecutive manner, wherein the respective other ultrasound transducers receive the emitted ultrasound pulse in each case. The distance between the ultrasound transducers and the shape of the connection layer can be determined on the basis of the time of flight, the amplitude or the phase of each of the emitted ultrasound pulses and received by the respective other remaining ultrasound transducers.

In a further embodiment, the ultrasound apparatus further comprises a light emitting device and a light detection device coupled to the processing unit, wherein the processing unit is further adapted to determine a shape of the connection layer on the basis of the light detected by the light detection device. This is a possibility to further improve the reliability of the detection of the shape of the connection layer.

In a further embodiment, the light emitting device and the light detection device are optically coupled by means of an optical fiber connected to the connection layer. This is a possibility to determine the shape of the connection layer with high precision.

In a further embodiment, the optical fiber is connected to the flexible and / or stretchable layer, wherein the processing unit is adapted to determine a strain of the optical fiber on the basis of the light detected by the light detection device and to determine the shape of the connection layer on the basis of the determined strain. This is a possibility to determine the shape of the connection layer precisely.

In a further embodiment, the processing unit is adapted to determine ultrasound image data on the basis of the ultrasound signal received from the plurality of ultrasound transducers and the determined distance between the ultrasound transducers and/or the determined shape of the connection layer. This is a possibility to utilize the ultrasound signals for ultrasound imaging, wherein the quality of the ultrasound image data can be improved on the basis of the determined distance between the ultrasound transducers and/or the determined shape of the connection layer.

As mentioned above, the ultrasound transducers which can be connected by means of the connection layer to the subject can provide a non-invasive and non-obstructive continuous and frequent monitoring of a medical parameter of the subject, wherein the evaluation and the interpretation of the different ultrasound signals received from the ultrasound transducers can be improved and a precise, robust and reliable measurement based on the ultrasound signals can be provided. Further, due to the connection layer, the ultrasound transducers can be attached individually to the subject and due to the measurement of the position of the ultrasound transducers with respect to each other and with respect to the anatomical feature of the subject, the measurement can be improved and an obstruction of a desired field of view e.g. by a bone, vessel, organ or other structures can be avoided.

BRIEF DESCRIPTION OF THE DRAWINGS

These and other aspects of the invention will be apparent from and elucidated with reference to the embodiment(s) described hereinafter. In the following drawings

Fig. 1 shows a schematic representation of an ultrasound apparatus in use for medical examination of a volume of a subject;
Fig. 2 shows a detailed representation of the ultrasound apparatus including a plurality of transducer elements;
Fig. 3 shows a detailed representation of the ultrasound apparatus including three transducer elements flexibly attached to each other;
Fig. 4 shows a schematic sectional view of the ultrasound apparatus including an ultrasound transducer and a connection layer;
Fig. 5 shows an embodiment of the ultrasound transducer including a strap connector;
Fig. 6 shows a sectional view of the embodiment of the ultrasound apparatus shown in Fig. 5;
Fig. 7 shows a sectional view of a further embodiment of the ultrasound transducer;
Fig. 8 shows a detailed top view of the ultrasound transducer including the plurality of ultrasound transducers attached to the connection layer;
Fig. 9 shows a sectional view of the ultrasound apparatus including a plurality of ultrasound transducers for determining a distance of the ultrasound transducers and/or a shape of the connection layer;
Fig. 10 shows a sectional view of an embodiment of the ultrasound apparatus including a light emitting device and a light detection device for determining a shape of the connection layer;
Fig. 11 shows a diagram of a shape and orientation function with respect to a Cartesian coordinate system; and
Fig. 12 shows a schematic top view of the ultrasound apparatus including an optical fiber for determining the shape of the connection layer.

DETAILED DESCRIPTION OF THE INVENTION

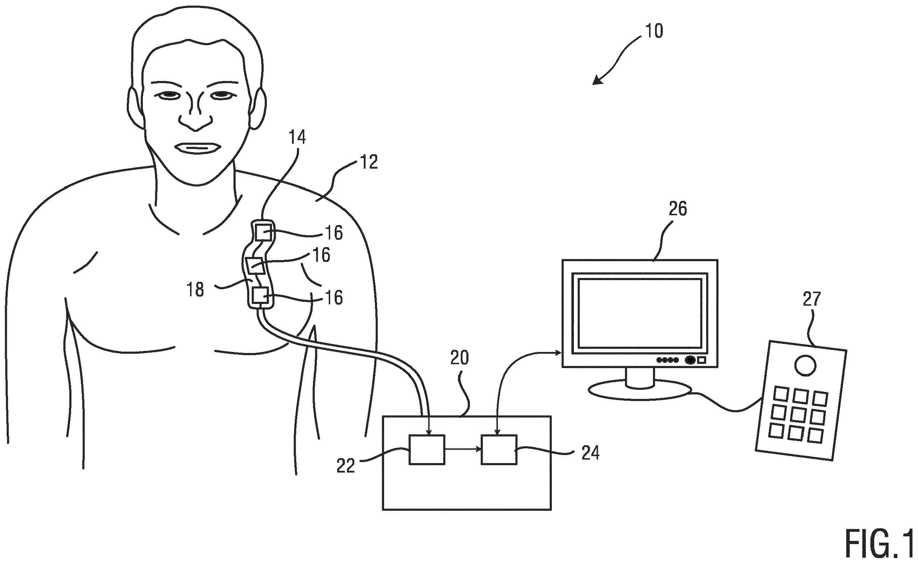

Fig. 1 shows a schematic illustration of an ultrasound apparatus for medical examination of a subject generally denoted by 10. The ultrasound apparatus 10 is applied to inspect a volume of an anatomical site, in particular an anatomical site of a subject 12 or a patient 12. The ultrasound apparatus 10 comprises an ultrasound transducer unit 14 including a plurality of ultrasound transducers 16 and a connection layer 18 for connecting the ultrasound transducers 16 to the subject 12. The connection layer 18 is a flexible and / or stretchable layer 18 for flexibly and expendably connecting the ultrasound transducers 16 to each other. The ultrasound transducer unit 14 may comprise two, three, four or more of ultrasound transducers 16 which are connected to the connection layer 18. The ultrasound transducers 16 comprises at least one ultrasound transducer element for emitting and receiving ultrasound waves and for providing a respective ultrasound signal on the basis of the ultrasound waves backscattered from the anatomical side of the subject 12. The ultrasound transducers 16 are electrically connected to each other by means of electrical wires and electrically connected to a central processing unit 20 for controlling the ultrasound transducers 16 and for receiving and evaluating the ultrasound signals from the ultrasound transducers 16. The central processing unit 20 comprises a control unit 22 for controlling the emission of the ultrasound waves and for receiving the respective ultrasound signals. The control unit 22 is connected to a processing unit 24 for receiving the ultrasound signal and for evaluating the ultrasound signals. The processing unit 24 is adapted to determine a relative position of the ultrasound transducers 16 to each other and/or a position of the ultrasound transducers 16 with respect to an anatomical feature of the subject 12 as described in the following. The processing unit 24 further determines at least one parameter which is indicative of a medical condition of the subject 12 on the basis of the ultrasound signals received from the ultrasound transducers 16. The central processing unit 20 and in particular the processing unit 24 is connected to a display device 26 for displaying results of the medical inspection to a user. The display device 26 may be connected to an input device 27 for controlling the ultrasound apparatus 10.

The ultrasound transducers 16 may comprise one transducer element or an array of transducer elements, wherein the ultrasound transducers 16 are in general coupled to the connection layer 18 which may be formed of a bendable and stretchable material so that a spacing between the ultrasound transducer 16 can be manually adjusted and the ultrasound transducer 16 can be placed individually at individually selected portions of the subject 12. This enables the user to inspect desired portions of the subject 12, in particular the lung, the heart, the aorta or the like or non-thoracic portions like the carotid, brachial, and femoral arteries, wherein the ultrasound transducers 16 are disposed at an intercostal position between the ribs or on / around the neck, arm, or leg of the subject 12 in order to achieve a non-obstructed view on an organ or a vessel to be examined. The processing unit 24 is adapted to determine a relative position of the ultrasound transducers 16 with respect to an anatomical feature e.g. a bone or an organ or other obstructing anatomical features in order to indicate if one or more of the ultrasound transducers 16 is disposed inappropriately at the subject 12 to provide a reliable measurement. The display device 26 may provide a corresponding warning to the operator.

The anatomical feature may be determined on the basis of artifacts like A-lines in the ultrasound signal or Doppler measurements for detection of arterial and/or venous vessels and assessment of perfusion. A well-positioned ultrasound transducer 16 can e.g. show the lung pleural line, which is associated to the artifacts of the A-lines. Hence, the proper position of the ultrasound transducer 16 can be automatically identified on the basis of the ultrasound signals.

For the case that the ultrasound transducer unit 14 is utilized for monitoring the lung of the subject 12, B-line artifacts can be utilized to determine the presence and the degree of a pulmonary edema which builds up from the bottom of the lung upwards. Also other lung conditions can be monitored which are associated with local lung water such as an infection, wherein the ultrasound transducers 16 can indicate the increase, the decrease or the migration of the local lung water. In another embodiment, the ultrasound transducer unit 14 can be adapted to measure cardiac, aortic, vascular or venous parameters. The ultrasound transducers 16 may be each equipped with a height meter or an accelerometer for determining a posture of the subject 12 which usually lead to a migration of lung water and alterations in hemodynamics due to the influence of the gravity. The height sensors can also be used to derive the degree, i.e. the height of the pulmonary edema based on which transducers find B-line artifacts and which transducers do not find B-line artifacts.

In an alternative embodiment, the ultrasound signals received from the ultrasound transducers 16 are used by the processing unit 24 to reconstruct and provide ultrasound image data. To reconstruct and provide the image data, the relative position of the ultrasound transducers 16 and the respective orientation of the individual ultrasound transducers 16 has to be registered. The relative position and the orientation of the ultrasound transducers 16 is determined on the basis of the ultrasound waves received by the ultrasound transducers 16 and evaluated by means of the processing unit 24. In an alternative embodiment, the shape of the connection layer 18 may be determined on the basis of light emitting devices and light detection devices attached or coupled to or integrated in the connection layer 18. On the basis of the so determined relative position of the ultrasound transducers 16 to each other and the individual orientation of the ultrasound transducers 16 an ultrasound image can be reconstructed. When the ultrasound transducers 16 form a vector or a line of elements, a 2D-image can be formed of the ultrasound signals as B-mode or plane wave compounding. If the ultrasound transducers 16 form a matrix of ultrasound transducers, a 3D-image can be formed.

The control unit 24 may control the ultrasound transducers 16 in order to focus the ultrasound beams emitted by the ultrasound transducers 16 to a region of interest on the basis of the detected anatomical features of the subject 12.

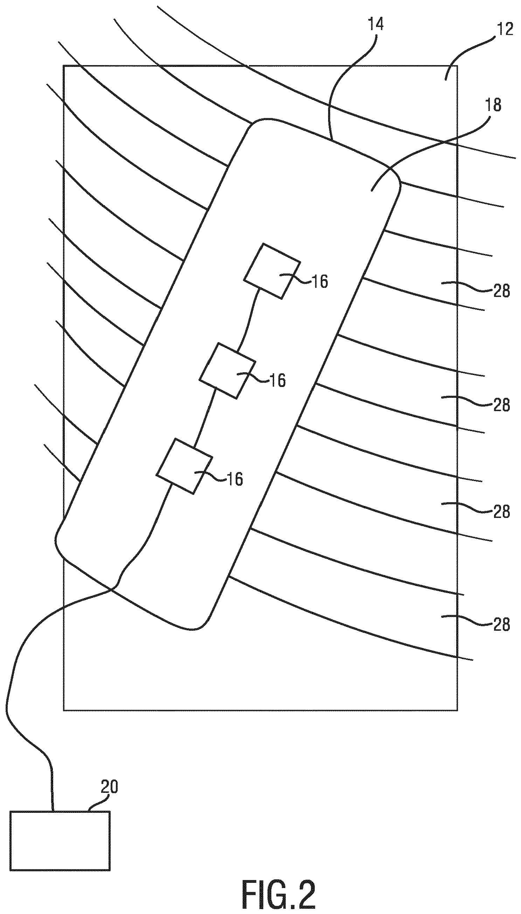

Fig. 2 shows a schematic view of the ultrasound transducer unit 14 attached to a site of the subject 12. The connection layer 18 is attached to the thorax of the subject 12 so that the ultrasound transducers 16 are disposed between ribs 28 of the subject 12. The connection layer 18 is attached to the skin of the subject 12 by means of a glue layer or an adhesive layer or a sticker. Each of the ultrasound transducers 16 may comprise one, two or multiple transducer elements for emitting and receiving ultrasound waves. The ultrasound transducers 16 may be capacitive micromachined ultrasonic transducer (CMUT), piezoelectric micromachined ultrasound transducer (PMUT) elements or C-mode, P-mode or polymer transducer such as PVDF transducer elements in order to emit and receive the ultrasound waves.

The processing unit 24 is adapted to determine on the basis of the ultrasound signal received from the ultrasound transducers 16 a relative position of the ultrasound transducers 16 with respect to anatomical features, in this particular case the ribs 28 so that an inappropriate position of the ultrasound transducer 16 at a rib 28 can be determined and indicated to the user by means of e.g. a warning signal. It should be noted that the anatomical feature may be any obstructing feature which is not of interest like bones, organs or vessels.

The processing unit 24 further determines a medical condition of the subject 12 on the basis of the ultrasound signals and in this particular case the condition of the lung, the heart, the aorta or other vessels and may determine a disease on the basis of the ultrasound signals.

Obstructing anatomical features are preferably determined on the basis of A-line artifact in the ultrasound signals. The medical condition of the subject 12, in this particular case pulmonary edema of the lung can be determined on the basis of B-line artifacts in the ultrasound signals.. Alternatively, for vascular purposes, Doppler signals are preferably used and in the case of the heart inspection other reconstructed signals or signal features can be utilized.

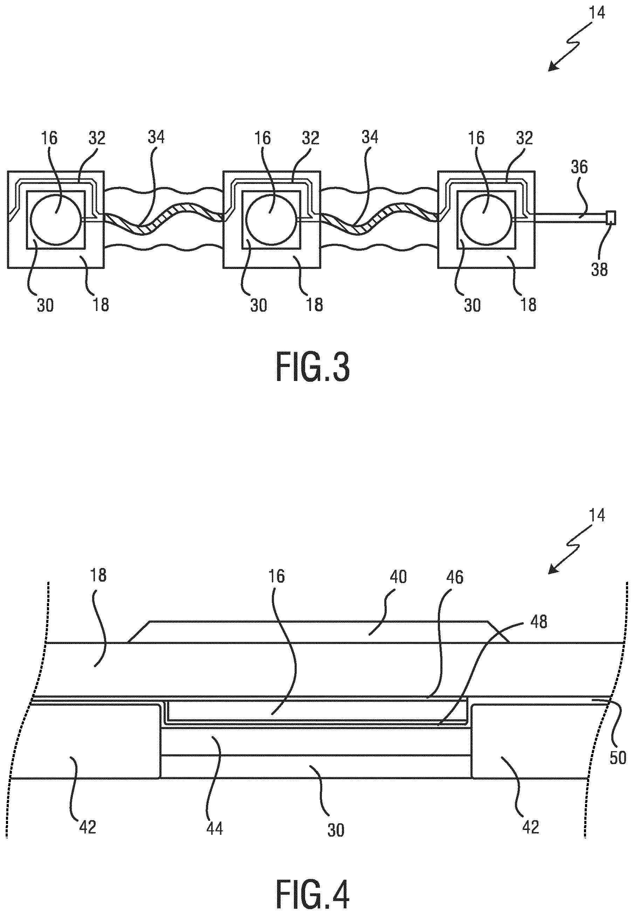

Fig. 3 shows a detailed diagram of the ultrasound transducer unit 14. The ultrasound transducers 16 are attached to the connection layer 18 by soldering, gluing or bonding, wherein the ultrasound transducers 16 are electrically connected by means of wires 32 which may be formed as printed wires 32 on the connection layer 18 or substrate 18. The transducer elements 16 can be attached to the skin of the subject 12 by means of a hydrogel layer 30. The ultrasound transducers 16 are connected to each other by means of the connection layer 18, which may be formed of a fabric, wherein conductive wires 34 are attached to the fabric in order to connect the printed wires 32 and the ultrasound transducers 16 to each other. The ultrasound transducer unit 14 is connected via a cable 36 and a connector 38 to the central processing unit 20. The part between the ultrasound transducers 16 can be cut into meander-like shape, for ensuring the flexibility and stretchability of the connection layer 18. All ultrasound transducers 16 are connected to the central electronics unit via a single connector. Another way to connect the ultrasound transducers 16 to the central processing unit 20 is to integrate flexible electronics into the connection layer 18 to make electrical contact between the central processing unit 20 and the transducers. In this case, the meandering of the patch between the tiles is not necessary.

The so flexibly connected ultrasound transducers 16 can be attached to individual portions of the subject 12 having a distance to each other, so that the ultrasound transducers 16 can be utilized for different medical examinations e.g. monitoring of the lung, the heart, vessels or other organs as mentioned above.

Fig. 4 shows a schematic sectional view of the ultrasound transducer unit 14. The ultrasound transducer 16 is attached to the connection layer 18, wherein the connection layer 18 is disposed on top of the ultrasound transducer element 16. On top of the connection layer 18 and the ultrasound transducer 16, a backing layer 40 is attached in order to absorb ultrasound energy of the ultrasound transducer 16 and to provide a pressure to the ultrasound transducer 16 to improve the attachment to the skin of the subject 12. Alternatively a pressure inducing layer may be attached to the backing layer 40 to provide the pressure to the ultrasound transducer 16. The backing layer 40 may have a thickness larger than the thickness of the pressure inducing layer. In an embodiment, the backing layer 40 may have a thickness at least double of the thickness of the pressure inducing layer. The ultrasound transducer 16 is surrounded by a base layer 42 to support the ultrasound transducer 16. The base layer 42 may be formed of felt or foam. Underneath the ultrasound transducer 16, a matching layer 44 is disposed, which is connected to the hydrogel layer 30 for providing a good ultrasound contact or coupling to the skin of the subject 12. The ultrasound transducer 16 is connected via electrodes 46, 48 to the printed wires 32, wherein the electrodes 46, 48 are isolated with respect to the matching layer 44 and the base layer 42 by means of an insulation layer 50.

The ultrasound transducers 16 can be soldered or bonded or glued with conductive adhesives onto a flexible printed circuit foil, e.g. polyethylene terephthalate (PET). The circuit on the both sides of the connection layer 18, except on the transducer area exposing to the skin, can be electrically insulated with a dielectric coating. A polymer layer filled with high density metal particles can be used as a top layer coating on the flexible layer opposite to the skin side of the ultrasound transducer unit 14, for damping ultrasound waves towards the opposite side of the skin. Each of the ultrasound transducers 16 can be addressed via a wire 32 on the connection layer 18, and, each of the ultrasound transducers 16 may comprise an analogue-to-digital converter.

The ultrasound transducer unit 14 may further comprise electronic devices. An additional central processing unit may be integrated that implements processing of RF data, i.e. the ultrasound signals including classification of lung, heart, and/or cardiovascular conditions or diseases and wireless communication. The ultrasound transducer unit 14 may also be wirelessly connected to the central processing unit 20. A wireless ultrasound transducer unit also requires an energy storage device such as a battery. A wired patch could offload all processing to an external unit by using a coax cable per transducer element. Alternatively, the ultrasound signals may be processed in the ultrasound transducer unit 14 and the ultrasound transducer unit 14 might be connected to another device with something as simple as a USB 3.0 cable.

Each of the ultrasound transducers 16 may be associated with an accelerometer and/or a height detector to determine a posture of the subject and/or a height of the respective ultrasound transducer 16 with respect to gravity and/or an anatomical feature of the subject 12.

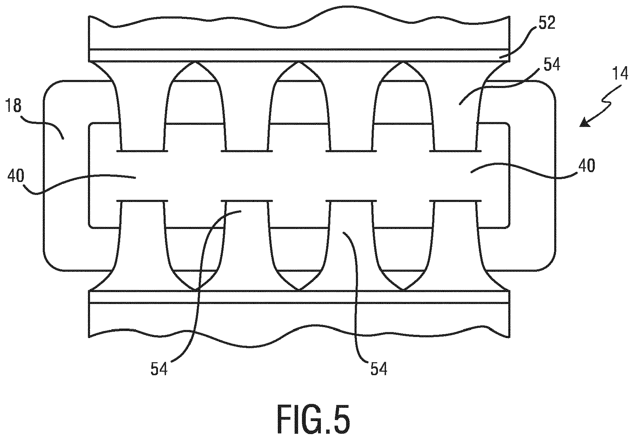

Fig. 5 shows a schematic top view of an embodiment of the ultrasound transducer unit 14 including a connection band 52, wherein the connection band 52 comprises a plurality of sub-bands 54 which are attachable to the backing layer 40 in order to absorb ultrasound energy and to improve the pressure by means of which the ultrasound transducers 16 are forced to the skin of the subject 12. The pressure inducing layer may be formed on top of the backing layer 40 and forms an additional spacer to induce local pressure. The connection band 52 can be worn around the thorax, an arm or a leg or at another site of the subject 12 in order to provide the respective contact force to the ultrasound transducers 16. This is a possibility to ensure the contact of the ultrasound transducers 16 at the skin of the subject 12, e.g. between the ribs 28 in the intercostal regions or at another portion of the subject 12.

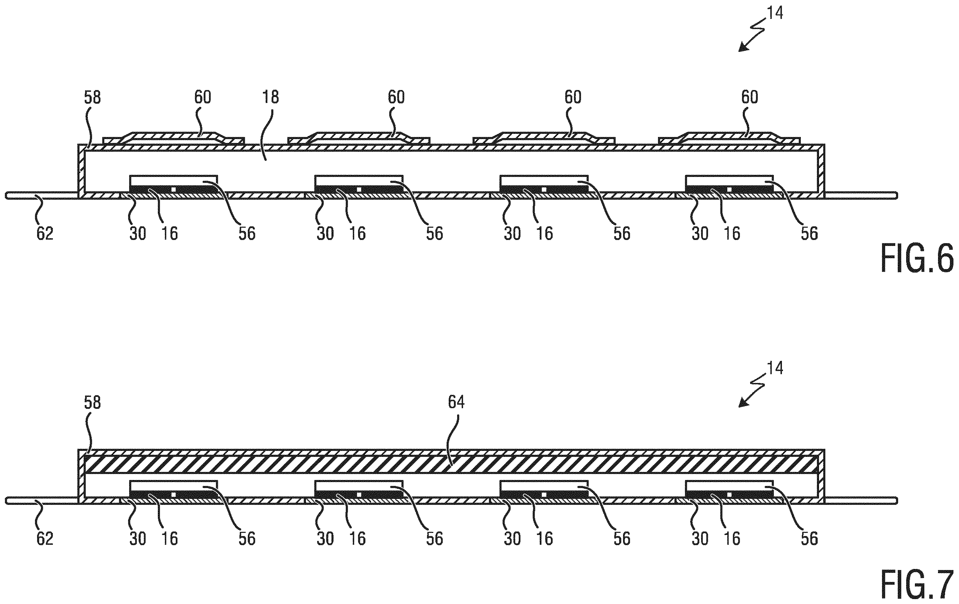

Fig. 6 shows a schematic sectional view of the ultrasound transducer unit 14. The ultrasound transducers 16 are attached to the hydrogel sheets 30 and covered by the connection layer 18. The ultrasound transducers 16 may also be attached to a substrate 56 including electronic devices for driving the ultrasound transducers 16. The flexible layer 18 may be covered by a cover layer 58, which may be formed of textile, wherein strap inserts 60 are attached to the cover layer 58 for supporting the sub-bands 54 which are attachable to the connection layer 18 in order to provide the improved pressure to the connection layer 18. The so arranged ultrasound transducer unit 14 can be properly fixed to the subject 12 including the necessary force, wherein the ultrasound transducers 16 can be individually disposed at certain positions due to the connection layer 18. The ultrasound transducers 14 may comprise an adhesive layer 62 in order to adhesively attach the ultrasound transducer unit 14 to the skin of the subject 12.

Fig. 7 shows a sectional view of an alternative embodiment of the ultrasound transducer unit 14. The ultrasound transducer unit 14 may further comprise a less flexible support layer 64 or a rigid layer 64 which is attached to the flexible layer 18 in order to provide a respective contact force to the ultrasound transducer 16 so that a proper contact can be provided.

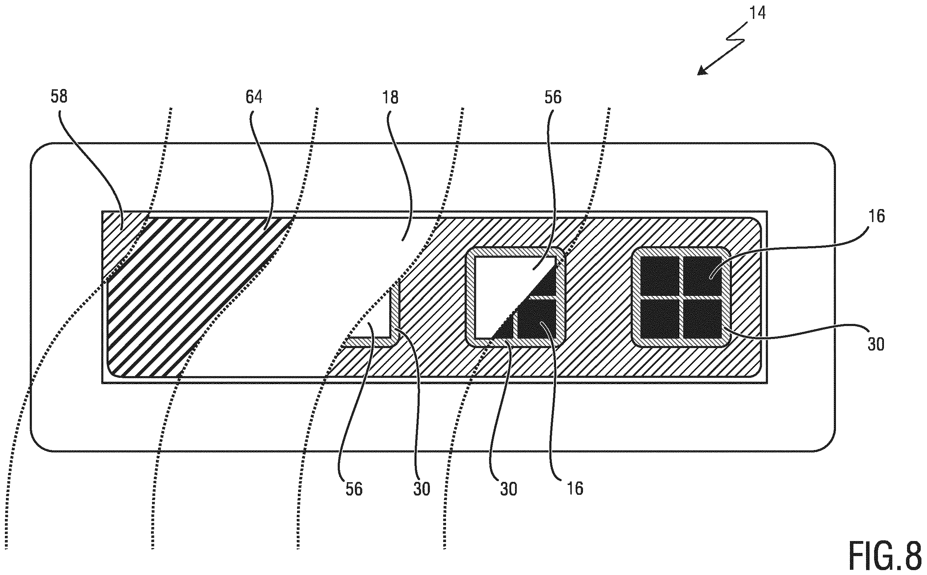

Fig. 8 shows a schematic top view of the ultrasound transducer unit 14, wherein the different layers are sectionally cut to show the different layers of the ultrasound transducer unit 14. The top surface of the ultrasound transducer unit 14 is formed by the cover layer 58. Underneath the cover layer 58, the less flexible or rigid layer 64 is disposed, wherein underneath the less flexible layer or rigid layer 64, the connection layer 18 is disposed. The cover layer 58 may be formed of textile or woven material. The transducer elements 16 may each comprise a substrate 56 including electronic devices for driving the ultrasound transducer 16. The ultrasound transducers 16 are attached to the hydrogel sheets 30 in order to provide a proper ultrasound transmission between the ultrasound transducer 16 and the subject 12. The flexible layer 18 including the ultrasound transducers 16 is surrounded by the adhesive layer 62 (not shown) in order to attach the ultrasound transducer unit 14 to the subject 12.

The ultrasound transducer unit 14 may use wired or wireless communication to transfer information regarding the placement of the ultrasound transducer unit 14 and the ultrasound transducers 16 and regarding the medical condition of the subject 12 to a user and/or a caregiver. As intermediate information, signals or signal quality information could be provided as feedback to user or caregiver. The user or caregiver can receive the information regarding the placement of the ultrasound transducer unit 14 and/or the ultrasound transducers 16 as well as (intermediate) measurement results on a phone, a headset, a near-to-eye-display, vital signs monitor or by visual signals provided by one or more LEDs or by acoustical signals provided by one or more speakers integrated in the ultrasound unit 14 in order to reduce the technical effort.

The respective status of the ultrasound transducer unit 14 can be summarized in a single signal or information. Alternatively the feedback could be provided of particular artifacts such as the presence or absence of A-line artifacts, the presence or absence of B-line artifacts, the presence or absence of lung sliding or on the basis of Doppler signals. An embodiment could use an LED to indicate a proper or inappropriate placement of the ultrasound transducers 16 and/or the medical condition of the subject 12. In a further embodiment, each of the ultrasound transducers 16 may comprise a plurality of LEDs to indicate to quality of the placement and the measurement result. As an example, a green LED could indicate a good placement or signal quality in combination with a healthy organ or vessel under this transducer, an orange LED could indicate that the ultrasound transducer 16 requires replacement, and a red LED could indicate a bad signal quality in combination with an unhealthy organ or vessel under this transducer.

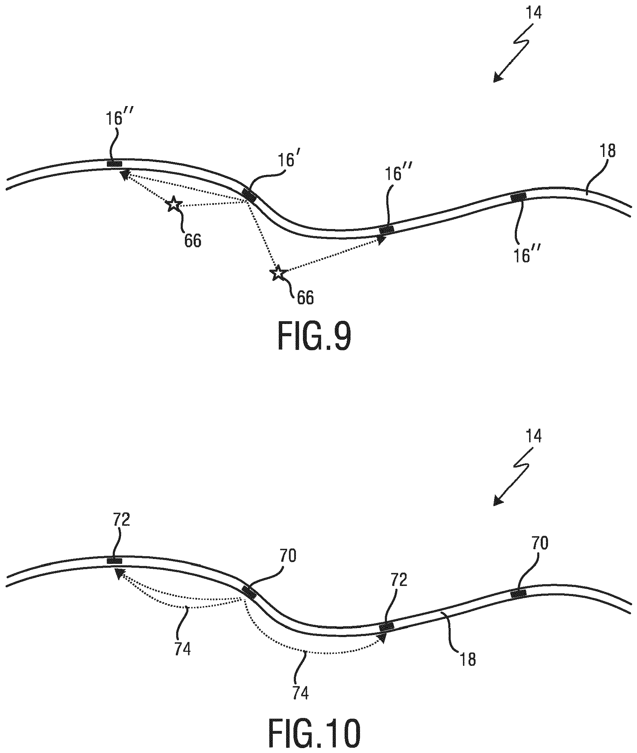

Fig. 9 shows an embodiment of the ultrasound transducer unit 14 including means to determine a relative position of the ultrasound transducers 16 to each other and/or a shape of the connection layer 18. The means for determining the relative position of the ultrasound transducers 16 and/or the shape of the connection layer 18 determines a distance between the ultrasound transducers 16, a relative angle between the ultrasound transducers 16 an angle of each ultrasound transducers 16 with respect to a normal direction, a position of the ultrasound transducers 16 with respect to the subject 12, an angle of ultrasound transducers 16 with respect to a gravity vector and / or an anatomical feature and/or a shape of the connection layer 18 to which the ultrasound transducers 16 are attached. On the basis of the relative position of the ultrasound transducers 16 to each other, a registration can be performed by means of the processing unit 24 in order to evaluate the ultrasound signals which can be utilized to form ultrasound image data on the basis of the ultrasound signals.

The shape of the connection layer 18 or the ultrasound transducer unit 14 can be determined using a spline interpolation wherein merely the local curvature in the direction perpendicular to the ultrasound transducers 16 is necessary. The resolution of the determined shape of the connection layer 18 is proportional to the density of the ultrasound transducers 16 and the necessary spatial resolution to determine the shape of the connection layer 18 depends on the flexibility or the rigidity of the connection layer or the ultrasound transducer unit 14 in general.

From the shape determination of the ultrasound transducer unit 14, the position of each of the ultrasound transducers 16 at the connection layer 18 or the ultrasound transducer unit 14 in a Cartesian coordinate system as a function of the curvy linear ultrasound transducer unit 14 coordinate can be determined e.g. by the formula:

$$r(u,v) = x(u,v)\hat{x} + y(u,v)\hat{y} + z(u,v)\hat{z}$$

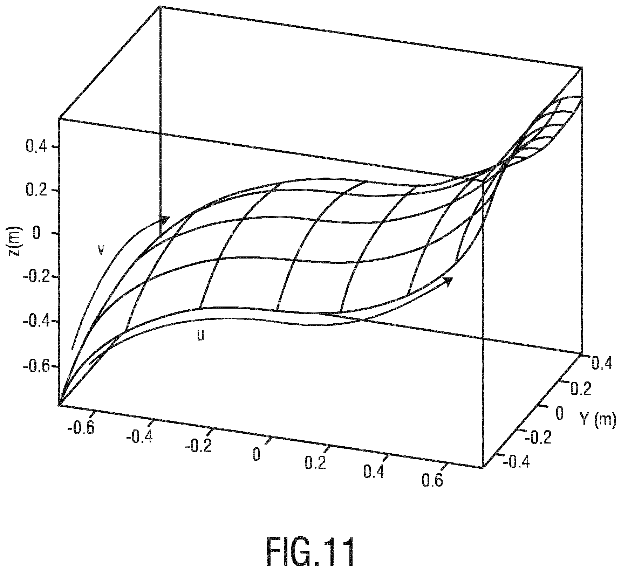

wherein r corresponds to the position of the ultrasound transducer unit 14 or the flexible layer 18 and u and v correspond to a curvy linear coordinates of the ultrasound transducer unit 14. It should be noted that this formula is one example and other functions can be used as well. An example for a function of a bended ultrasound transducer unit 14 in the Cartesian coordinate system is shown in Fig. 11.

In Fig. 9, one embodiment of the ultrasound transducer unit 14 including ultrasound transducers 16 for determining the relative position of the ultrasound transducers 16 to each other and the corresponding shape of the connection layer 18 is schematically shown.

The ultrasound transducer unit 14 comprises one ultrasound transducer 16' which is adapted to emit an ultrasound pulse signal, wherein the other ultrasound transducers 16" receives the pulse signal from the emitting ultrasound transducer 16'. On the basis of the time of flight, the amplitude or a phase parameter of the so transmitted ultrasound pulse signal, the relative position of each of the ultrasound transducer 16" with respect to the emitting ultrasound transducer 16' can be determined. The ultrasound pulse signal may be received directly through the subject 12 or may be backscattered from a specific anatomical feature 66 of the subject 12 as shown in Fig. 9. The corresponding path through which the ultrasound pulse signal is transmitted from the emitting ultrasound transducer 16' to the receiving ultrasound transducer 16" depends on the curvature of the connection layer 18.

The emission or transmission of the ultrasound pulse signal should be repeated for different of the ultrasound transducers 16 in order to determine the shape of the connection layer 18 or the shape of the ultrasound transducer unit 14.

Alternatively the shape of the ultrasound transducer unit 14 or the connection layer 18 may be determined by additional ultrasound transducers which are acoustically coupled, e.g. glued to the connection layer 18. The ultrasound transducers 16 induce and measure surface acoustic waves through the ultrasound transducer unit 14 or the connection layer 18. In general the detection of the shape on the basis of the surface acoustic waves through the flexible layer 18 is more robust, since the shape detection depends only on the ultrasound transducer unit 14 and not on the tissue of the subject 12.

Fig. 10 shows an alternative embodiment of the ultrasound transducer unit 14 for determining the shape of the connection layer 18 or the ultrasound transducer unit 14. The ultrasound transducer unit 14 comprises a plurality of light emitters 70 and a plurality of light detectors 72 which are integrated in the connection layer 18 or the ultrasound transducer unit 14 in general. The light emitters 70 and the light detectors 72 may be disposed at all or a subset of the ultrasound transducers 16 or at locations between the ultrasound transducers 16. The light detectors 72 measure light 74 which is emitted from the light emitters 70 and transmitted due to sub-surface scattering in the tissue of the subject 12. In the case that the ultrasound transducer unit 14 is bended around the subject 12, the light signal is distorted. When the ultrasound transducer unit 14 is curved around the subject 12, the detected light is increased due to the direct optical path and when the ultrasound transducer unit 14 is curved in the other way, the amount of detected light is reduced due to a longer and indirect path of the light.

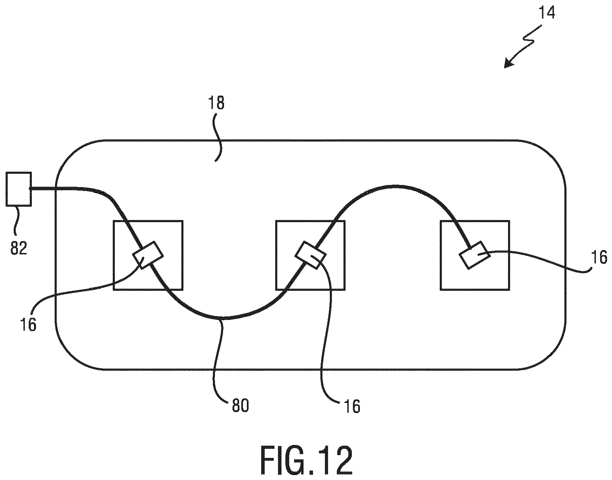

Fig. 12 shows an alternative embodiment of the ultrasound transducer unit 14 including an optical fiber 80 which is integrated in the connection layer 18 and connected to an optical interrogator unit 82. The optical interrogator unit 82 is designed to measure spatially resolved scattering along the optical fiber 80 by techniques such as Optical Frequency Domain Reflectometry or optical readout of inscribed fiber Bragg gratings. Such a measurement can be used to locally determine strain in the optical fiber 80. From such a strain measurement the shape of the ultrasound transducer unit 14 or the connection layer 18 can be determined. As such, the shape of the ultrasound transducer unit 14 is determined on the basis of the detected distribution of light along the optical fiber 80. The optical fiber 80 may be integrated or connected in parallel to the electric wire 34.

Similarly, ultrasound could be utilized instead of light, wherein the light guide 80 is replaced by a gel-filled conducting path between the ultrasound transducer 16 or additional ultrasound transducers, wherein the gel-filled conducting path acoustically couples the ultrasound transducer 16 or the additional ultrasound transducer through by means of which the ultrasound waves can be transmitted. The gel-filled path can be a narrow tube with ultrasound-insulated walls or ultrasound-reflecting walls. When the path deforms, then the ultrasound transmission characteristics change and this can be measured again via the time of flight measurement, an amplitude or a phase measurement.

Hence, the shape of the ultrasound transducer device 14 can be determined precisely on the basis of ultrasound emission and detection or light emission and detection so that a registering of the ultrasound transducers 16 can be performed and physiological data can be derived from the ultrasound signals.

Computer program code for carrying out the methods of the present invention by execution on the processing unit 24 may be written in any combination of one or more programming languages, including an object oriented programming language such as Java, Smalltalk, C++ or the like and conventional procedural programming languages, such as the "C" programming language or similar programming languages. The program code may execute entirely on the processing unit 24 as a stand-alone software package, e.g. an app, or may be executed partly on the processing unit 24 and partly on a remote server. In the latter scenario, the remote server may be connected to the head-mountable computing device through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the processing unit 24, such that the instructions create means for implementing the functions/acts specified in the flowchart and/or block diagram block or blocks. These computer program instructions may also be stored in a computer-readable medium that can direct the cardiopulmonary resuscitation guidance system including the portable computing device to function in a particular manner.

The computer program instructions may, for example, be loaded onto the portable computing device to cause a series of operational steps to be performed on the portable computing device and/or the server, to produce a computer-implemented process such that the instructions which execute on the portable computing device and/or the server provide processes for implementing the functions/acts specified in the flowchart and/or block diagram block or blocks. The computer program product may form part of a patient monitoring system including a portable computing device.

While the invention has been illustrated and described in detail in the drawings and foregoing description, such illustration and description are to be considered illustrative or exemplary and not restrictive; the invention is not limited to the disclosed embodiments. The invention is defined in the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. Thus, there may be provided a computer program product downloadable from a communications network and/or stored on a computer readable medium and/or microprocessor-executable medium wherein the computer program product comprises computer program code instructions, which when executed by at least one processor, implement a method according to a proposed embodiment.

Any reference signs in the claims should not be construed as limiting the scope.

## Claims

1. Ultrasound apparatus (10) for medical examination of a subject (12), comprising:

    a plurality of ultrasound transducers (16) for emitting and receiving ultrasound waves and for providing different ultrasound signals on the basis of the ultrasound waves,
    a flexible and / or stretchable connection layer (18), attachable to the subject, wherein the ultrasound transducers (16) are coupled to the connection layer (18), and
    a processing unit (24) coupleable to the ultrasound transducers (16) for receiving the ultrasound signals and for determining at least one parameter on the basis of the ultrasound signals derived from the ultrasound waves backscattered from an anatomical feature of the subject (12), wherein the at least one parameter is indicative of a position of the ultrasound transducers (16) with respect to the anatomical feature of the subject and/or indicative of a medical condition of the subject,

wherein the processing unit (24) is adapted to determine at least one parameter indicative of a relative position of the ultrasound transducers (16) to each other and / or a shape of the connection layer (18),

**characterized in that** the ultrasound transducers (16) are adapted for inducing and measuring surface acoustic waves transmitted through the connection layer (18) or a layer attached to the connection layer (18), and **in that** the processing unit is adapted for determining a distance between the ultrasound transducers (16) and/or for determining the shape of the connection layer on the basis of the surface acoustic waves transmitted through the connection layer (18) or a layer attached to the connection layer (18).

2. Ultrasound apparatus as claimed in claim 1, wherein the position of the ultrasound transducers (16) with respect to the anatomical feature is determined on the basis of at least one of A-line detection, B-line detection, amplitude detection, or Doppler signal detection.

3. Ultrasound apparatus as claimed in claim 1, further comprising an accelerometer, wherein the processing unit is adapted to determine a posture of the subject on the basis of a signal received from the accelerometer.

4. Ultrasound apparatus as claimed in claim 1, further comprising a plurality of height detectors associated to the ultrasound transducers (16) for providing height signals corresponding to a height of the respective ultrasound transducer with respect to anatomical features of the subject, wherein the processing unit is adapted to determine a posture of the subject and/or changes of the posture of the subject on the basis of the height signals.

5. Ultrasound apparatus as claimed in claim 1, further comprising a beam forming unit (22) for focusing the ultrasound waves emitted by the ultrasound transducers, and wherein the beam forming unit is adapted to focus the ultrasound waves emitted by the ultrasound transducers (16) on the basis of the relative position of the ultrasound transducers (16) to each other and/or on the basis of the position of the ultrasound transducers (16) with respect to the anatomical feature of the subject.

6. Ultrasound apparatus as claimed in claim 1, wherein the ultrasound transducers (16) have a distance to each other and wherein the processing unit is adapted to determine a distance between the ultrasound transducers (16) on the basis of the ultrasound waves received by the ultrasound transducers.

7. Ultrasound apparatus as claimed in claim 1, wherein the processing unit is adapted to determine a shape of the flexible layer on the basis of the ultrasound waves received by the ultrasound transducers (16).

8. Ultrasound apparatus as claimed in claim 6 or 7, wherein the processing unit (24) is further adapted to determine the distance between the ultrasound transducers (16) and/or the shape of the connection layer (18) based on the backscattered ultrasound waves received by the ultrasound transducers (16).

9. Ultrasound apparatus as claimed in any of claims 6 to 8, wherein the processing unit (24) is further adapted to determine the distance between the ultrasound transducers (16) and/or the shape of the connection layer (18) based on the ultrasound waves emitted by one of the ultrasound transducers.

10. Ultrasound apparatus as claimed in claim 1, further comprising a light emitting device (70) and a light detection device (72), wherein both devices are integrated in the connection layer (18) and coupled to the processing unit, wherein the processing unit is further adapted to determine the shape of the connection layer (18) on the basis of the light detected by the light detection device.

11. Ultrasound apparatus as claimed in claim 10, wherein an optical fiber (80) is connected to the connection layer (18), wherein the processing unit is adapted to determine a strain of the optical fiber on the basis of the light detected by the light detection device and to determine the shape of the connection layer (18) on the basis of the determined strain.

12. Ultrasound apparatus as claimed in any of claims 6 to 10, wherein the processing unit is adapted to determine ultrasound image data on the basis of the ultrasound signals received from the ultrasound transducers (16), wherein said ultrasound signals are derived from the ultrasound waves backscattered from an anatomical feature of the subject (12), and the determined distance between the ultrasound transducers (16) and/or the determined shape of the connection layer (18).

13. Method for medical examination of a subject, comprising the steps of:

emitting and receiving ultrasound waves by means of a plurality of ultrasound transducers attachable via a flexible and / or stretchable connection layer to the subject and providing a plurality of different ultrasound signals on the basis of the ultrasound waves received by the plurality of ultrasound transducers,

- determining at least one parameter on the basis of the ultrasound signals derived from the ultrasound waves backscattered from an anatomical feature of the subject (12), wherein the at least one parameter is indicative of a position of the ultrasound transducers with respect to the anatomical feature of the subject and / or indicative of a medical condition of the subject,

determining at least one parameter indicative of a relative position of the ultrasound transducers to each other and / or a shape of the connection layer,

**characterized in that**
determining a distance between the ultrasound transducers on the basis of the ultrasound waves received by the ultrasound transducers and/or determining the shape of the connection layer is performed on the basis of the ultrasound waves received by the ultrasound transducers which are surface acoustic waves transmitted through the connection layer or a layer attached to the connection layer.

14. Method as claimed in claim 13, comprising the step of determining the position of the ultrasound transducers with respect to the anatomical feature on the basis of at least one of A-line detection, B-line detection, amplitude detection, or Doppler signal detection.

15. A computer program product for medical examination of a subject, the computer program product comprising computer-readable program code downloadable from a communications network, or storable on, or stored on a computer-readable storage medium, which computer-readable program code, when run on a computer causes the computer to perform the following steps:

controlling a plurality of ultrasound transducers, for emitting and receiving ultrasound waves, attachable via a flexible and / or stretchable connection layer to the subject and providing a plurality of different ultrasound signals on the basis of the ultrasound waves received by the plurality of ultrasound transducers,
determining at least one parameter on the basis of the ultrasound signals, said signals derived from the ultrasound waves backscattered from an anatomical feature of the subject (12) and received by the ultrasound transducers, wherein the at least one parameter is indicative of a position of the ultrasound transducers with respect to an anatomical feature of the subject and / or indicative of a medical condition of the subject,
determining at least one parameter indicative of a relative position of the ultrasound transducers to each other and / or a shape of the connection layer,

**characterized in that**

determining a distance between the ultrasound transducers on the basis of the ultrasound waves received by the ultrasound transducers and/or determining the shape of the connection layer is performed on the basis of the ultrasound waves received by the ultrasound transducers which are surface acoustic waves transmitted through the connection layer or a layer attached to the connection layer.

**Patentansprüche**

1. Ultraschallgerät (10) zur medizinischen Untersuchung einer Person (12), umfassend:

eine Vielzahl von Ultraschallwandlern (16) zum Aussenden und Empfangen von Ultraschallwellen und zum Bereitstellen verschiedener Ultraschallsignale auf der Basis der Ultraschallwellen,
eine flexible und/oder dehnbare Verbindungsschicht (18), die an der Person angebracht werden kann, wobei die Ultraschallwandler (16) mit der Verbindungsschicht (18) gekoppelt sind, und
eine Verarbeitungseinheit (24), die mit den Ultraschallwandlern (16) gekoppelt werden kann, um die Ultraschallsignale zu empfangen und um mindestens einen Parameter auf der Basis der Ultraschallsignale zu bestimmen, die von den Ultraschallwellen abgeleitet werden, welche von einem anatomischen Merkmal der Person (12) rückgestreut werden, wobei der mindestens eine Parameter eine Position der Ultraschallwandler (16) in Bezug auf das anatomische Merkmal der Person und/oder eine medizinische Verfassung der Person angibt, wobei die Verarbeitungseinheit (24) dafür ausgelegt ist, mindestens einen Parameter zu bestimmen, der eine relative Position der Ultraschallwandler (16) zueinander und/oder eine Form der Verbindungsschicht (18) angibt,

**dadurch gekennzeichnet, dass** die Ultraschallwandler (16) dafür ausgelegt sind, akustische Oberflächenwellen zu induzieren und zu messen, die durch die Verbindungsschicht (18) oder eine an der Verbindungsschicht (18) angebrachte Schicht übertragen werden, und dadurch, dass die Verarbeitungseinheit dafür ausgelegt ist, einen Abstand zwischen den Ultraschallwandlern (16) und/oder die Form der Verbindungsschicht auf der Basis der durch die Verbindungsschicht (18) oder eine an der Verbindungsschicht (18) angebrachte Schicht übertragenen akustischen Oberflächenwellen zu bestimmen.

2. Ultraschallgerät nach Anspruch 1, wobei die Position der Ultraschallwandler (16) in Bezug auf das anatomische Merkmal auf der Basis von mindestens einem von A-Linien-Detektion, B-Linien-Detektion, Amplitudendetektion oder Dopplersignal-Detektion bestimmt wird.

3. Ultraschallgerät nach Anspruch 1, ferner umfassend einen Beschleunigungsmesser, wobei die Verarbeitungseinheit dafür ausgelegt ist, eine Haltung der Person auf der Basis eines von dem Beschleunigungsmesser empfangenen Signals zu bestimmen.

4. Ultraschallgerät nach Anspruch 1, ferner umfassend eine Vielzahl von zu den Ultraschallwandlern (16) gehörigen Höhendetektoren zum Bereitstellen von Höhensignalen, die einer Höhe des jeweiligen Ultraschallwandlers in Bezug auf anatomische Merkmale der Person entsprechen, wobei die Verarbeitungseinheit dafür ausgelegt ist, eine Haltung der Person und/oder Haltungsänderungen der Person auf der Basis der Höhensignale zu bestimmen.

5. Ultraschallgerät nach Anspruch 1, ferner umfassend eine Strahlformungseinheit (22) zum Fokussieren der durch die Ultraschallwandler ausgesendeten Ultraschallwellen, und wobei die Strahlformungseinheit dafür ausgelegt ist, die durch die Ultraschallwandler (16) ausgesendeten Ultraschallwellen auf der Basis der relativen Position der Ultraschallwandler (16) zueinander und/oder auf der Basis der Position der Ultraschallwandler (16) in Bezug auf das anatomische Merkmal der Person zu fokussieren.

6. Ultraschallgerät nach Anspruch 1, wobei die Ultraschallwandler (16) einen Abstand zueinander haben und wobei die Verarbeitungseinheit dafür ausgelegt ist, einen Abstand zwischen den Ultraschallwandlern (16) auf der Basis der durch die Ultraschallwandler empfangenen Ultraschallwellen zu bestimmen.

7. Ultraschallgerät nach Anspruch 1, wobei die Verarbeitungseinheit dafür ausgelegt ist, eine Form der flexiblen Schicht auf der Basis der durch die Ultraschallwandler (16) empfangenen Ultraschallwellen zu bestimmen.

8. Ultraschallgerät nach Anspruch 6 oder 7, wobei die Verarbeitungseinheit (24) ferner dafür ausgelegt ist, den Abstand zwischen den Ultraschallwandlern (16) und/oder die Form der Verbindungsschicht (18) basierend auf den durch die Ultraschallwandler (16) empfangenen rückgestreuten Ultraschallwellen zu bestimmen.

9. Ultraschallgerät nach einem der Ansprüche 6 bis 8, wobei die Verarbeitungseinheit (24) ferner dafür ausgelegt ist, den Abstand zwischen den Ultraschallwandlern (16) und/oder die Form der Verbindungsschicht (18) basierend auf den durch einen der Ultraschallwandler ausgesendeten Ultraschallwellen zu bestimmen.

10. Ultraschallgerät nach Anspruch 1, ferner umfassend eine lichtemittierende Vorrichtung (70) und eine Lichtdetektionsvorrichtung (72), wobei beide Vorrichtungen in die Verbindungsschicht (18) integriert und mit der Verarbeitungseinheit gekoppelt sind, wobei die Verarbeitungseinheit ferner dafür ausgelegt ist, die Form der Verbindungsschicht (18) auf der Basis des durch die Lichtdetektionsvorrichtung detektierten Lichts zu bestimmen.

11. Ultraschallgerät nach Anspruch 10, wobei eine optische Faser (80) mit der Verbindungsschicht (18) verbunden ist, wobei die Verarbeitungseinheit dafür ausgelegt ist, eine Dehnung der optischen Faser auf der Basis des durch die Lichtdetektionsvorrichtung detektierten Lichts zu bestimmen und die Form der Verbindungsschicht (18) auf der Basis der bestimmten Dehnung zu bestimmen.

12. Ultraschallgerät nach einem der Ansprüche 6 bis 10, wobei die Verarbeitungseinheit dafür ausgelegt ist, Ultraschallbilddaten auf der Basis der von den Ultraschallwandlern (16) empfangenen Ultraschallsignale, wobei die genannten Ultraschallsignale von den Ultraschallwellen abgeleitet werden, die von einem anatomischen Merkmal der Person (12) rückgestreut werden, und dem bestimmten Abstand zwischen den Ultraschallwandlern (16) und/oder der bestimmten Form der Verbindungsschicht (18) zu bestimmen.

13. Verfahren zur medizinischen Untersuchung einer Person, umfassend die folgenden Schritte:

Aussenden und Empfangen von Ultraschallwellen mittels einer Vielzahl von Ultraschallwandlern, die über eine flexible und/oder dehnbare Verbindungsschicht an der Person angebracht werden können, und Bereitstellen einer Vielzahl von verschiedenen Ultraschallsignalen auf der Basis der Ultraschallwellen, die durch die Vielzahl von Ultraschallwandlern empfangen werden,
Bestimmen von mindestens einem Parameter auf der Basis der Ultraschallsignale, die von den Ultraschallwellen abgeleitet werden, welche von einem anatomischen Merkmal der Person (12) rückgestreut werden, wobei der mindestens eine Parameter eine Position der Ultraschallwandler in Bezug auf das anatomische Merkmal der Person und/oder eine medizinische Verfassung der Person angibt, Bestimmen von mindestens einem Parameter, der eine relative Position der Ultraschallwandler zueinander und/oder eine Form der Verbindungsschicht angibt,

**dadurch gekennzeichnet, dass**

das Bestimmen eines Abstands zwischen den Ultraschallwandlern auf der Basis der durch die Ultraschallwandler empfangenen Ultraschallwellen und/oder das Bestimmen der Form der Verbindungsschicht auf der Basis der Ultraschallwellen erfolgt, die durch die Ultraschallwandler empfangen werden, wobei es sich um akustische Oberflächenwellen handelt, die durch die Verbindungsschicht oder eine an der Verbindungsschicht angebrachte Schicht übertragen werden.

14. Verfahren nach Anspruch 13, umfassend den Schritt des Bestimmens der Position der Ultraschallwandler in Bezug auf das anatomische Merkmal auf der Basis von mindestens einem von A-Linien-Detektion, B-Linien-Detektion, Amplitudendetektion oder Dopplersignal-Detektion.

15. Computerprogrammprodukt zur medizinischen Untersuchung einer Person, wobei das Computerprogrammprodukt computerlesbaren Programmcode umfasst, der von einem Kommunikationsnetzwerk heruntergeladen werden kann oder auf einem computerlesbaren Speichermedium gespeichert werden kann oder gespeichert ist, wobei der computerlesbare Programmcode, wenn er auf einem Computer ausgeführt wird, den Computer veranlasst, die folgenden Schritte durchzuführen:

Steuern einer Vielzahl von Ultraschallwandlern zum Aussenden und Empfangen von Ultraschallwellen, die über eine flexible und/oder dehnbare Verbindungsschicht an der Person angebracht werden können, und Bereitstellen einer Vielzahl von verschiedenen Ultraschallsignalen auf der Basis der Ultraschallwellen, die durch die Vielzahl von Ultraschallwandlern empfangen werden,
Bestimmen von mindestens einem Parameter auf der Basis der Ultraschallsignale, wobei die genannten Signale von den Ultraschallwellen abgeleitet werden, welche von einem anatomischen Merkmal der Person (12) rückgestreut werden, und durch die Ultraschallwandler empfangen werden, wobei der mindestens eine Parameter eine Position der Ultraschallwandler in Bezug auf ein anatomisches Merkmal der Person und/oder eine medizinische Verfassung der Person angibt,
Bestimmen von mindestens einem Parameter, der eine relative Position der Ultraschallwandler zueinander und/oder eine Form der Verbindungsschicht angibt, **dadurch gekennzeichnet, dass**
das Bestimmen eines Abstands zwischen den Ultraschallwandlern auf der Basis der durch die Ultraschallwandler empfangenen Ultraschallwellen und/oder das Bestimmen der Form der Verbindungsschicht auf der Basis der Ultraschallwellen erfolgt, die durch die Ultraschallwandler empfangen werden, wobei es sich um akustische Oberflächenwellen handelt, die durch die Verbindungsschicht oder eine an der Verbindungsschicht angebrachte Schicht übertragen werden.

**Revendications**

1. Appareil à ultrasons (10) pour un examen médical d'un sujet (12), comprenant :

une pluralité de transducteurs à ultrasons (16) pour émettre et recevoir des ondes ultrasonores et pour fournir différents signaux ultrasonores sur la base des ondes ultrasonores,
une couche de connexion flexible et/ou étirable (18), pouvant être fixée au sujet, dans lequel les transducteurs à ultrasons (16) sont couplés à la couche de connexion (18), et
une unité de traitement (24) pouvant être couplée aux transducteurs à ultrasons (16) pour recevoir les signaux ultrasonores et pour déterminer au moins un paramètre sur la base des signaux ultrasonores dérivés des ondes ultrasonores rétrodiffusées d'un élément anatomique du sujet (12), dans lequel l'au moins un paramètre est indicatif d'une position des transducteurs à ultrasons (16) par rapport à l'élément anatomique du sujet et/ou indicatif d'un état médical du sujet,
dans lequel l'unité de traitement (24) est conçue pour déterminer au moins un paramètre indicatif d'une position relative des transducteurs à ultrasons (16) les uns par rapport aux autres et/ou d'une forme de la couche de connexion (18),
**caractérisé en ce que** les transducteurs à ultrasons (16) sont conçus pour induire et mesurer des ondes acoustiques de surface transmises par l'intermédiaire de la couche de connexion (18) ou d'une couche fixée à la couche de connexion (18), et **en ce que** l'unité de traitement est conçue pour déterminer une distance entre les transducteurs à ultrasons (16) et/ou pour déterminer la forme de la couche de connexion sur la base des ondes acoustiques de surface transmises par l'intermédiaire de la couche de connexion (18) ou d'une couche fixée à la couche de connexion (18).

2. Appareil à ultrasons selon la revendication 1, dans lequel la position des transducteurs à ultrasons (16) par rapport à l'élément anatomique est déterminée sur la base d'au moins l'une parmi une détection de ligne A, une détection de ligne B, une détection d'amplitude, ou une détection de signal Doppler.

3. Appareil à ultrasons selon la revendication 1, comprenant en outre un accéléromètre, dans lequel l'unité de traitement est conçue pour déterminer une posture du sujet sur la base d'un signal reçu de l'accéléromètre.

4. Appareil à ultrasons selon la revendication 1, comprenant en outre une pluralité de détecteurs de hauteur associés aux transducteurs à ultrasons (16) pour fournir des signaux de hauteur correspondant à une hauteur du transducteur à ultrasons respectif par rapport à des éléments anatomiques du sujet, dans lequel l'unité de traitement est conçue pour déterminer une posture du sujet et/ou des changements de la posture du sujet sur la base des signaux de hauteur.

5. Appareil à ultrasons selon la revendication 1, comprenant en outre une unité de formation de faisceau (22) pour focaliser les ondes ultrasonores émises par les transducteurs à ultrasons, et dans lequel l'unité de formation de faisceau est conçue pour focaliser les ondes ultrasonores émises par les transducteurs à ultrasons (16) sur la base de la position relative des transducteurs à ultrasons (16) les uns par rapport aux autres et/ou sur la base de la position des transducteurs à ultrasons (16) par rapport à l'élément anatomique du sujet.

6. Appareil à ultrasons selon la revendication 1, dans lequel les transducteurs à ultrasons (16) ont une distance les uns par rapport aux autres et dans lequel l'unité de traitement est conçue pour déterminer une distance entre les transducteurs à ultrasons (16) sur la base des ondes ultrasonores reçues par les transducteurs à ultrasons.

7. Appareil à ultrasons selon la revendication 1, dans lequel l'unité de traitement est conçue pour déterminer une forme de la couche flexible sur la base des ondes ultrasonores reçues par les transducteurs à ultrasons (16).

8. Appareil à ultrasons selon la revendication 6 ou 7, dans lequel l'unité de traitement (24) est en outre conçue pour déterminer la distance entre les transducteurs à ultrasons (16) et/ou la forme de la couche de connexion (18) sur la base des ondes ultrasonores rétrodiffusées reçues par les transducteurs à ultrasons (16).

9. Appareil à ultrasons selon l'une quelconque des revendications 6 à 8, dans lequel l'unité de traitement (24) est en outre conçue pour déterminer la distance entre les transducteurs à ultrasons (16) et/ou la forme de la couche de connexion (18) sur la base des ondes ultrasonores émises par l'un des transducteurs à ultrasons.

10. Appareil à ultrasons selon la revendication 1, comprenant en outre un dispositif émetteur de lumière (70) et un dispositif de détection de lumière (72), dans lequel l'un et l'autre des dispositifs sont intégrés dans la couche de connexion (18) et couplés à l'unité de traitement, dans lequel l'unité de traitement est en outre conçue pour déterminer la forme de la couche de connexion (18) sur la base de la lumière détectée par le dispositif de détection de lumière.

11. Appareil à ultrasons selon la revendication 10, dans lequel une fibre optique (80) est connectée à la couche de connexion (18), dans lequel l'unité de traitement est conçue pour déterminer une contrainte de la fibre optique sur la base de la lumière détectée par le dispositif de détection de lumière et pour déterminer la forme de la couche de connexion (18) sur la base de la contrainte déterminée.

12. Appareil à ultrasons selon l'une quelconque des revendications 6 à 10, dans lequel l'unité de traitement est conçue pour déterminer des données d'image ultrasonore sur la base des signaux ultrasonores reçus des transducteurs à ultrasons (16), dans lequel lesdits signaux ultrasonores sont dérivés des ondes ultrasonores rétrodiffusées à partir d'un élément anatomique du sujet (12), et de la distance déterminée entre les transducteurs à ultrasons (16) et/ou de la forme déterminée de la couche de connexion (18).

13. Procédé d'examen médical d'un sujet, comprenant les étapes consistant à :

émettre et recevoir des ondes ultrasonores au moyen d'une pluralité de transducteurs à ultrasons pouvant être fixés par l'intermédiaire d'une couche de connexion flexible et/ou étirable au sujet et fournissant une pluralité de signaux ultrasonores différents sur la base des ondes ultrasonores reçues par la pluralité de transducteurs à ultrasons,

- déterminer au moins un paramètre sur la base des signaux ultrasonores dérivés des ondes ultrasonores rétrodiffusées à partir d'un élément anatomique du sujet (12), dans lequel l'au moins un paramètre est indicatif d'une position des transducteurs à ultrasons par rapport à l'élément anatomique du sujet et/ou indicatif d'un état médical du sujet,

déterminer au moins un paramètre indicatif d'une position relative des transducteurs à ultrasons les uns par rapport aux autres et/ou d'une forme de la couche de connexion, **caractérisé en ce que**

la détermination d'une distance entre les transducteurs à ultrasons sur la base des ondes ultrasonores reçues par les transducteurs à ultrasons et/ou la détermination de la forme de la couche de connexion sont effectuées sur la base des ondes ultrasonores reçues par les transducteurs à ultrasons qui sont des ondes acoustiques de surface transmises par l'intermédiaire de la couche de connexion ou d'une couche fixée à la couche de connexion.

**14.** Procédé selon la revendication 13, comprenant l'étape de détermination de la position des transducteurs à ultrasons par rapport à l'élément anatomique sur la base d'au moins l'une parmi une détection de ligne A, une détection de ligne B, une détection d'amplitude, ou une détection de signal Doppler.

**15.** Produit programme informatique pour un examen médical d'un sujet, le produit programme informatique comprenant un code de programme lisible par un ordinateur, téléchargeable à partir d'un réseau de communications, ou stockable sur, ou stocké sur, un support de stockage lisible par un ordinateur, lequel code de programme lisible par un ordinateur, lorsqu'il est exécuté sur un ordinateur amène l'ordinateur à effectuer les étapes suivantes :

commander une pluralité de transducteurs à ultrasons, pour émettre et recevoir des ondes ultrasonores, pouvant être fixés par l'intermédiaire d'une couche de connexion flexible et/ou étirable au sujet et fournissant une pluralité de signaux ultrasonores différents sur la base des ondes ultrasonores reçues par la pluralité de transducteurs à ultrasons,

déterminer au moins un paramètre sur la base des signaux ultrasonores, lesdits signaux dérivés des ondes ultrasonores rétrodiffusées à partir d'un élément anatomique du sujet (12) et reçues par les transducteurs à ultrasons, dans lequel l'au moins un paramètre est indicatif d'une position des transducteurs à ultrasons par rapport à un élément anatomique du sujet et/ou indicatif d'un état médical du sujet,

déterminer au moins un paramètre indicatif d'une position relative des transducteurs à ultrasons les uns par rapport aux autres et/ou d'une forme de la couche de connexion, **caractérisé en ce que**

la détermination d'une distance entre les transducteurs à ultrasons sur la base des ondes ultrasonores reçues par les transducteurs à ultrasons et/ou la détermination de la forme de la couche de connexion sont effectuées sur la base des ondes ultrasonores reçues par les transducteurs à ultrasons qui sont des ondes acoustiques de surface transmises par l'intermédiaire de la couche de connexion ou d'une couche fixée à la couche de connexion.

FIG.1

FIG.2

FIG.3

FIG.4

FIG.5

FIG.6

FIG.7

58    64    18    56    16

16

30

56    30    30    16

EP 3 349 663 B1

FIG.8

FIG.9

FIG.10

FIG.11

FIG.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5485841 A
- US 20130079641 A1